# EUROPEAN PATENT APPLICATION

(11) **EP 1 661 533 A1**
(43) Date of publication of application: **31.05.2006**
(21) Application number: 04786432.7
(22) Date of filing: 18.08.2004
(51) Int. Cl.: A61F 9/007

(54) **METHOD FOR EYE REFRACTIVE SURGERY AND DEVICE FOR IMPLANTING AN INTRAOCULAR REFRACTIVE LENS**

(30) Priority: 21.08.2003 RU 2003125614
(71) Applicant: Khuray, Aslan Ramazanovich, Moscow, 117437 (RU)
(72) Inventor: KHURAY, Aslan Ramazanovich, Moscow, 117437 (RU); LATYPOV, Ilyas Amirovich, Moscow, 117588 (RU)
(74) Representative: Zech, Stefan Markus
(86) International application number: PCT/RU2004/000324
(87) International publication number: WO 2005/018515

(57) **Abstract**

The invention relates to medicine, in particular to ophtalmosurgery. The inventive method for an eye refractive surgery consists in delating the pupil by mydriatics, anaesthetising, and subsequently in incising the cornea. Afterwards, the anterior chamber is filled with a low-molecular-weight viscoelastic material and a refractive lens is implanted with the aid of a cannula which is connected to a source of vacuum ranging from 40 to 60 mm hg. For this purpose, said refractive lens is fixed to the midele between the lens edge and the boundary of the optical part thereof by means of the working cannula end and is bent thereon. Afterwards, the refractive lens is introduced into the cornea incision by the top of the bend and arranged in the posterior chamber of the eye. Vacuum is removed, the refractive lens being detached from the cannula and extracted from the anterior chamber of the eye. The inventive device for implanting the intraocular refractive lens comprises a cannula provided with a stop. Said cannula comprises a working funnel-shaped end face having an oval cross section, is connected to the body of a vacuum syringe and made of a low-pressure polyethylene or stainless steel, the working end thereof being coated with polyurethane methacrylate.

## Description

An invention relates to medicine, its ophthalmologic surgery branch, and may be used in ophthalmologic operations for implanting intraocular refractive lens to the anterior chamber of the eye.

A method and a tool (pneumatic magazine) for installation of a contact lens (RF patent No. 2,200,517, published 20.03.2003, cl. A 61 F 9/00). The pneumatic magazine contains rubber ball syringe and a cone-shaped suction cup for holding the contact lens. Said suction cup cone contains a rod for limiting the "absorbable" surface of the lens. As said ball syringe is finger compressed, either a vacuum for lens attachment to the suction cup or a pressure for detaching it after applying to retina is formed.

However, the refraction surgery requires much finer e.g. ultra-compact and accurate sizes, which would allow for operating in a strictly limited space e.g. said anterior chamber of the eye, where the depth (about 3 mm) and slight movements cause irreversible and dreadful consequences for the crystalline lens and cornea endothelium.

A tool is also known - PRL INJECTOR (Ciba Vision Surgical, Dulith, USA), inside which cartridge a lens is folded and then by pushing is implanted to the anterior chamber of the eye. The main disadvantage of said injector is a possibility of lens rotation upside down inside the cartridge, which will cause its improper location. Therefore, the concept of the lens location - distancing from the anterior capsule of the crystal - will be disturbed e.g. the lens adheres to the crystal, which will cause disturbance of the crystal metabolism and further lenticular opacity (complicated cataract, refer to *Refraction Surgery and Ophthalmology,* 2003, vol. 3(4), pp. 15 - 18 (in Russian); *"Implantation of facial posterior chambers intraocular lenses (PRL, Ciba Vision) for ametropia correction",* D.D. Dement'ev, E.V. Shestykh, and T.L. Fadeikina, Moscow Research Ophthalmologic Center "New Sight", Moscow, Russia).

A method and a tool for episcleral filling of retina (RF patent No. 2,197,208, published on 27.01.2003, cl. A 61 F 9/007) are known. Said tool contains elastic jaws, which operating components form a cylinder, through which the filling compound is squeezed out by a plunger to the retina. Said plunger is fixed in the jaws, equipped with a limit stop. The tool provides for a reliable intake of the filling compound, reduces the operation time and injuries of the globe of the eye tissues.

However, proper introduction of said intraocular refractive lens requires fixing it by a working tool, which must carry it through and cut tunnel in the cornea preserving the entity of the lens and causing no traumas to the surrounding tissue.

Thus, the most appropriate technical solution is a method and a tool for implanting intraocular lenses, described in RF patent No. 2,189,303, published on 27.09.2002, cl. A 61 F 9/007, representing a prototype of suggested method and tool.

The known tool contains an operating part for fixing and applying said lens, made from biological inert plastic material. It is V-shaped at its distal end for introducing a needle with a thread, which is to fix the lens. The operating part is equipped with a handle for advancing. The method realized in case of using said tool includes cutting cornea by a sclerocorneal tunnel 3, 5 or 4 mm deep, then introducing said operation tool to said posterior chamber, introducing said needle with a suture No. 9 or 10made from nonabsorbable monophilic material - prolene. After maximal introduction of the needle to the operating part, the tool with the needle and the suture is withdrawn from the globe of the eye.

The suture is connected to said intraocular lens, which then by drawing the suture is implanted to an iridociliary furrow, fixed by the suture indraught from the sclera side, and knotted under the surface fragment of the sclera. Said technical solution allows for reducing traumatism of the procedure and duration of the implantation operation.

A tool is also known, applied to ophthalmologic operations for PRL implantation, the Dement'ev pincer, which operating part is made from two branches possessing working surfaces facing one another. These surfaces fix the lens by a haptic not touching the optic part. The pincer is equipped with a handle for out-in traverse of the operating part. The guaranteed gap at connecting the branch surfaces excludes damaging of the optical part of PRL lens during implantation. However, the application of said pincer may induce severe consequences, if a surgeon makes even minor mistakes: they are damages of the anterior capsule of the crystal and cornea endothelium. At the reverse movement of the pincer PRL may fall back.

The technical task, to solve which a number of inventions is aimed, is decrease of the eye tissue traumatism in the operation zone and improvement of the operation quality.

This technical problem was solved by a method that according to the current invention, including cornea cutting, fixing and implantation of said refractive lens by said operating tool, after anesthesia and extending the pupil by mydriatic compounds cornea is cut (Clear cornea - 3 mm). After that the anterior chamber is filled by a viscoelastic compound of low molecular weight and said refractive lens is implanted using the cannula connected to 40 - 60 mmHg vacuum source. For this purpose, said refractive lens is fixed by the working face of the cannula at the middle between the lens edge and the optic are border, when the edge is bent to the cannula face. Then the top of the lens bend is injected into the cornea cut, and the lens is set in the posterior chamber of the eye. When vacuum is eliminated, the cannula is detached from said refractive lens, and said cannula is removed from the anterior chamber.

Said technical task was also resolved by the composition of the tool for said refractive lens implantation, which, according to said invention, has the operating part for fixing and carrying said refractive lens through the cornea cut to the anterior chamber of the eye and the handle. In said tool, the operating part is shaped as a cannule representing a tube bent at the angle of 120 - 130°, extending oval-shaped cross-section behind the bending and trumpet-shaped working face, 0.4 - 0.6 mm long and ellipse-shaped cross-section with the minor and major axes equal 0.8 - 0.9 and 1.8 - 2.0 mm, respectively. Said cannule is made from biologically inert material with the tensile strength 27 MPa or higher. The edge and the internal surface of said trumpet-shaped working face of the cannule are covered a polymer with tensile strength 2.9 - 3 MPa. Said handle for cannule manipulation represents an evacuating syringe, connected to the cannule via a socket.

Moreover, said cannule is made from LDPE and stainless steel, and said covering material is polyurethane methacrylate.

The technical result of realization of said method and the tool is as follows:
- said intraocular refractive lens to be implanted is not mechanically fixed (compressed) by pincer faces, which eliminates danger of the lens damaging;
- said intraocular refractive lens cannot be turned upside down inside the cartridge, whence improper implantation is eliminated;
- a possibility of traumas for both intraocular refractive lens to implanted and the surrounding tissues of the eye is reduced;
- the operation time is reduced compared with other methods, because implantation is complete in a single slight movement;
- said method simplicity allows for adapting this operation to the broadest range of ophthalmologists;
- retrieval of said tool handle is not expensive and should be made once. This excludes any sterilization, and danger of the patient infecting is reduced.
   As compared with the prototype, it has been found that said method differs by the vacuum fixing of the lens (40 - 60 mmHg), bending of the lens edge and using it in the lens advancing by said tunnel until said lens is properly located and its surface is disconnected from said cannule face after removal of the vacuum. Said tool has an original construction of the operating part, also original parameters of said cannule material and sizes, and the moving handle implemented as a vacuum syringe.

### Disclosure of invention

Said lens is fixed to said cannule face due to vacuum (40 - 60 mmHg deep) that excludes any mechanical damaging of said lens. Said cannule is of miniature size, determined experimentally. Various shapes of said cannule working face have been tested, for example, a polyethylene tube reinforced by a stainless steel tube, with cylindrical or oval-shaped soft working face.

Experimentally, it has been found that LDPE mechanical parameters re satisfactory for preserving the bent shape of the cannule tube and the strength of the whole structure, required during the operation. This material trademark has been selected among analogous polymeric materials, such as poly(methyl methacrylate) (PMMA) collagen, hydrogel. Said material has the following parameters: tensile at break - 400 MPa, tensile strength - 27 MPa, elasticity modulus at bending - 550 - 700 MPa. As said tool and technique of said intraocular refractive lens implantation was developed, the cannule made from stainless steel showed equal results. Meanwhile, it has been found that said working face must be shaped as a hollow paddle (a flattened funnel) with a hole enough for said lens suction by vacuum and the external surface, which causes no damage to the lens bending at its advancing. The material selected for the working face is polyurethane methacrylate as tensile strong as 2.9 - 3.0 MPa, covering the edge and the internal surface of the V-shaped face of said cannule. Geometrical sizes were selected experimentally with respect to the size of the operation cut and the lens to be implanted. Said tool has no mechanisms, which would be moved inside the eye. Detachment of said cannule from said intraocular refractive lens requires no manipulations in the anterior chamber of the eye. Smooth and straight surface of said cannule eliminates the danger of damaging tissues during dragging the tool out from the eye. At the implantation, there are no contacts of said cannule and the eye tissues, and any contact between said cannule and the front surface of the crystal capsule is eliminated. Since said cannule is moved in parallel to the crystal of the eye and there is a limiter behind said cannule bending, any crystal semiluxation is eliminated. Said cannule body trespassing through the cornea cut prevents bleeding of the anterior chamber of the eye that allows for preserving the anterior chamber of the eye operating. Said lens turning upside down id eliminated, because said lens is fixed. Said lens can be visually monitored during the operation. Any opportunity of unwilling re-implanting of said lens during reverse movement of said cannule is eliminated. Said tool is inexpensive one-shot tool that excludes sterilization procedures and reduces danger of personal infecting of the current patient. The unique handle of the tool with said evacuating syringe makes the tool rather compact and suitable for a surgeon and his hand.

The technique suggested cut time of operation and avoids any complications, because the operating tool fixes said lens so that the cannule is enveloped in the lens, and any contact with the anterior capsule of the crystal and cornea endothelium is completely avoided.

According to said invention, the method for refraction surgery of the eye, the intraocular refractive lens implantation is implemented as follows. After local anesthesia 3 mm long cut of cornea with preliminary medicated medryase is made. Thereafter, the anterior chamber is filled with a viscoelastic compound of low molecular weight and said intraocular refractive lens is implanted using said cannule, evacuated to 40 - 60 mmHg. Said lens is fixed by the trampet-shaped face in the middle between the lens edge and the border of the optical area. The edge of said lens is bent on the cannule face and then by the bend top the lens is introduced into the cornea cut. Thereafter, said lens is fixed in the anterior chamber of the eye, vacuum is removed and the cannule is detached from said intraocular refractive lens, and said cannule is removed from the anterior chamber.

The existing complexity of the surgical operation at implantation of the intraocular refractive lens limits the possibility of its wide application to the ophthalmologic practice. Said invention allows for simplifying the operation technique and ring-fence both a patient and a surgeon from possible complications.

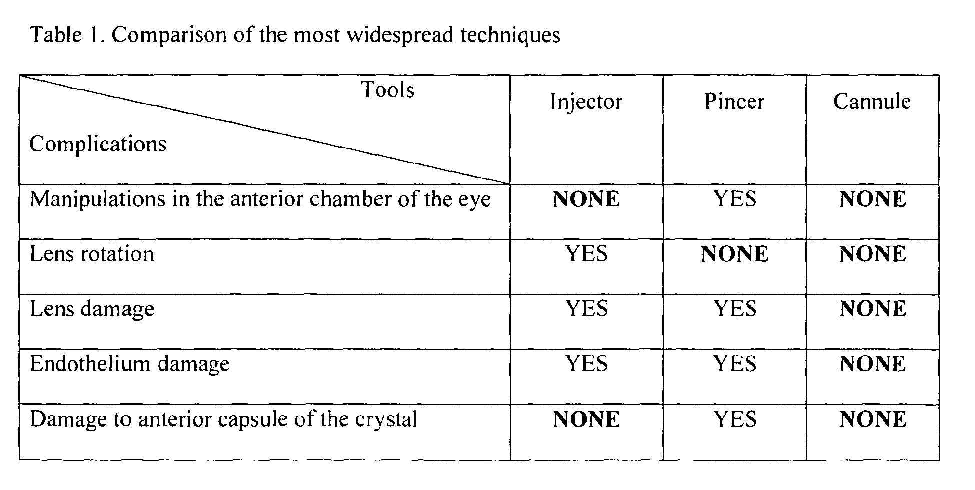

The conditions of said intraocular refractive lens fixing by said cannule must be so that the place of fixing must be located directly at the haptic center, said lens must be bent on the cannule edge in order to intensify the contact between the lens and cannule owing to resistance during passing the tunnel.

**Table2. Lens samples tested**

| Lens model | Length, mm | Width, mm | Type | Number of implanted lenses |
|---|---|---|---|---|
| 100 | 10.8 | 6 | Myopic | 5 |
| 101 | 11.3 | 6 | Myopic | 5 |
| 200 | 10.6 | 6 | Hypermetropic | 5 |

Said models were tested by vacuum sucking on by said cannule in different sites. Several said tests performed at 30 mmHg vacuum indicate significant lens cut-in or sucking in of its surface by the cannule. For 70 mmHg vacuum, exfoliation of said lens surface from the cannule face was observed at its movement. Thus, 40 - 60 mmHg vacuum is optimal.

The experiments with imprints of said cannule face on said lens surface allowed for selecting optimal sizes of said cannule face flattened as the oval. A combination of cannule strength (avoiding its twisting and bending), the channel size, lens preservation, easiness of penetration through the cornea cut and taking it out of the tunnel.

The evacuation syringe connected to said cannule was tested for a possibility of making 40 - 60 mmHg vacuum. A cavity before the piston was connected to a pressure-rarefaction sensor and a limiter, a fixer for the piston movement was selected in order to make the structure and functioning of the vacuum syringe providing a surgeon reliability of all parameters.

Surgical operations were performed in the Moscow Research Ophthalmologic Center "New Sight". The clinical example: patient M (female), 32 years old, entered into the hospital with a diagnosis: high myopia OU, anisometropia, OD sph. -13.75 = cyl. -2.5 ax 180. OS sph. -0.5 = cyl. -1.5 ax 180, to whom LASIK of the left eye was performed on 0.7.07.2003. The next operation on the right eye - intraocular refractive lens implantation - was performed on 14.07.2003.

The operation run: the pupil is medicamentally extended. After local anesthesia the cornea is 12 h direction cut by 3 mm. Said viscoelastic compound with low molecular mass is injected into the anterior chamber of the eye. Using efacuation, said intraocular refractive lens is fixed to the cannule face. Using said cannule said lens is implanted to the anterior chamber of the eye, then vacuum is removed, cannule is removed from said chamber by the reverse motion. Using an applicator said haptic of the lens is set properly under the iris of the eye. The anterior chamber of the eye is rinsed with a physiological solution. A pupil-narrowing compound (myocol) is injected to the anterior chamber and, using the pincer and micro-iris scissors, peripheral iridoectomy is performed. At the site of the cornea cut BSS solution for non-suture adaptation of the wound edges is injected to the stroma. The conjunctival sac is dropped by an antibiotic solution.

The next day after the operation the eye is still, cornea is transparent, and the anterior chamber contains clear fluid. The pupil actively reacts on the light influence, no contact with the front surface of the crystal is observed; the fundus of the eye shows pink reflux. The refraction equals OD sph. -0.75 = cyl. -1.5 ax 180. Thus, 13.00 diopters were obtained.

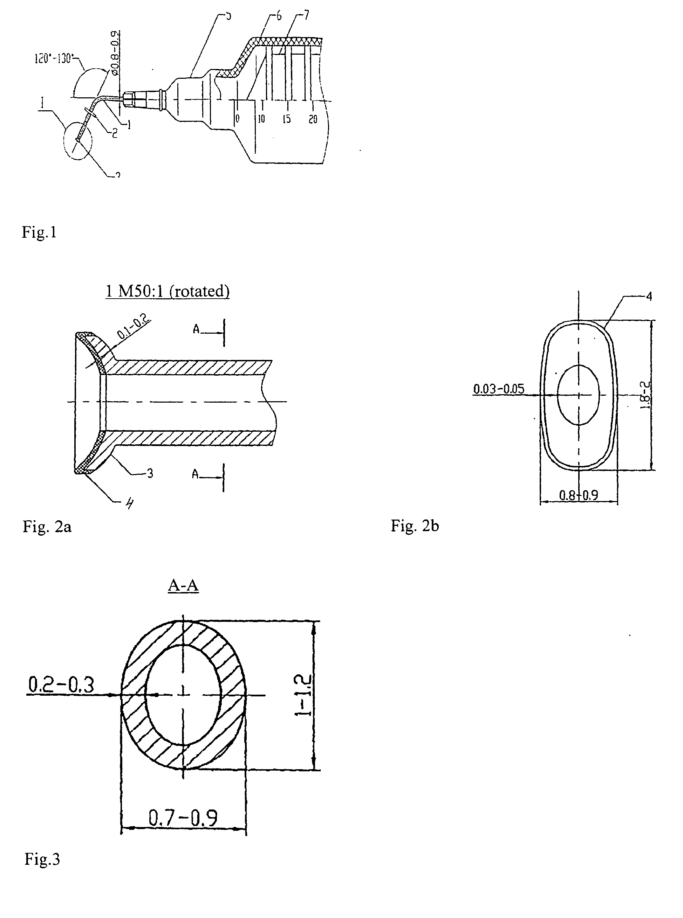

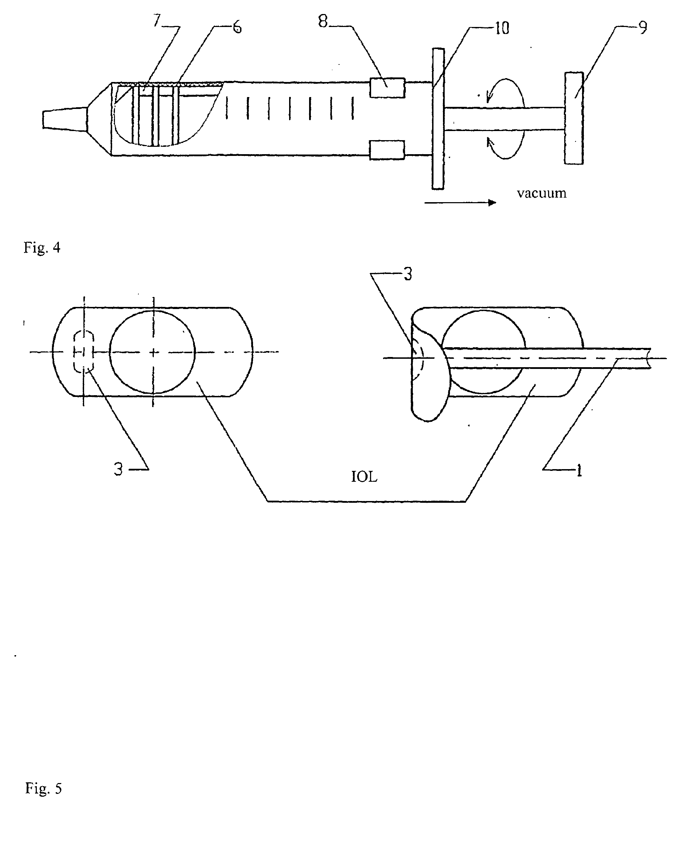

The invention is explained by blueprint in Figure 1 representing the overall view of the tool, and by Figures 2 and 3, in which sections and sectional elevations are shown. Figures 4 and 5 present the syringe and the lens, respectively. The cannule 1 is equipped with the limiter 2 behind the bending and has the V-shaped working face 3 of the oval section 4, which is covered by polyurethane methacrylate inside. The cannule shaped as a tube 0.6 - 0.9 mm in diameter is bent at 120 - 130°. The cannule is connected to the case 6 of the evacuating syringe via the trumpet 5. Inside the case 6 (Figure 5) the syringe is equipped with the piston 7, which movements are limited by the stopper 8, shaped as a bayonet. The piston is equipped with a plunger 9 with a button, the case 6 - by a collar 10 for comfortable handing. The vacuum source may also be a phacoemulsificator connected to the cannule trumpet through a hollow handle. Figure 4 shows the vacuum syringe blueprint.

The tool operates as follows: it fixes said intraocular refractive lens at the cannule face using vacuum and implantation of said intraocular refractive lens to the anterior chamber of the eye (Figure 5).

## Claims

1. A method for refraction surgery of the eye, including cutting of the cornea, fixing and implantation of the refractive lens by a working tool, which is specific by extension of the pupil of the eye by mydriatic agents and a cut of the cornea after anesthesia. Thereafter, the anterior chamber of the eye is filled with a viscoelastic compound with low molecular weight and then the refractive lens is implanted with the help of said cannule, connected to the source of 10 - 600 mmHg vacuum. For this purpose, the refractive lens is fixed by the working face of the cannule at the middle between the lens edge and the border of the optic area, with the edge bent on front upper part of the cannule face. Then by the top of the bending the refractive lens is introduced into the cornea cut and set in the posterior chamber of the eye. Thereafter, vacuum is removed and the cannule is detached from the refractive lens and taken off the anterior chamber of the eye by the reverse movement.

2. A tool for implantation of the refractive lens containing a working component for fixing and introducing the refractive lens through the cornea cut into the chamber of the eye and a handle, specific by the cannule-shaped working component, which represents a tube bent by 110 - 160°, equipped with a concave limiter, 2 - 8 mm in diameter and 1 - 3 mm wide after the bending, round or oval section, the working end of which (0.2 - 2.0 mm long) is oval-shaped with minor and major axes equal 0.6 - 0.9 mm and 1.5 - 2.5 mm, respectively. Hence, the cannule is made from a biologically inert material with tensile strength 27 MPa or higher. Said handle is shaped as a hollow tube with 1 - 5 mm internal and 4 - 10 mm external diameters, covered by a cellular knurl, is designed for moving said cannule, and connection to the vacuum source via an elastic mains, connected to said cannule via a trumpet.

3. The tool of claim 2, wherein the said cannule is shaped as a tube made from low pressure polyethylene (LDPE), reinforced by a tube from stainless steel.
